# EUROPEAN PATENT APPLICATION

(11) **EP 1 241 266 A1**
(43) Date of publication of application: **18.09.2002**
(21) Application number: 00979970.1
(22) Date of filing: 07.12.2000
(51) Int. Cl.: C12Q 1/68, C12N 15/09

(54) **METHOD OF DETECTING NUCLEOTIDE POLYMORPHISM**

(30) Priority: 10.12.1999 JP 35183799; 10.07.2000 JP 2000208794
(71) Applicant: Toyo Boseki Kabushiki Kaisha, Osaka 530-0004 (JP)
(72) Inventor: AONO, Toshiya Research Center of Toyo Boseki K.K., Shiga 520-0243 (JP); TAKARADA, Yutaka Research Center Toyo Boseki K.K., Shiga 520-0243 (JP); SEGAWA, Masaya Research Center of Toyo Boseki K.K, Shiga 520-0243 (JP); YOSHIGA, Satoko Research Center Toyo Boseki K.K, Shiga520-0243 (JP)
(74) Representative: VOSSIUS & PARTNER
(86) International application number: JP0008657
(87) International publication number: WO01042498

(57) **Abstract**

A method of detecting variation or polymorphism in a nucleic acid sequence, which is useful particularly in diagnosing a hereditary disease, analyzing nucleotide polymorphism, etc., and primers to be used therein. More particularly speaking, this method comprises treating a primer for wild type and one or two primers for variant with DNA polymerase either simultaneously or separately, and detecting the nucleotide polymorphism contained in the nucleic acid sample by judging whether or not the primers are extended, or whether or not the primers are amplified with a reverse primer. In this method, the 3'-end second bases of the primer for wild type and one or two primers for variant correspond to respective nucleotides anticipated in the nucleotide polymorphism site. Further, at least one of the bases from the 3'-end third position to the 5'-end is substituted by a base not complementary to the base in the chain hybridized with the primer in the chromosome or fragment. Furthermore, the not complementary base as described above differs from primer to primer.

## Description

### TECHNICAL FIELD

The present invention relates to a method of identifying variations or polymorphisms in nucleic acid sequences, and to primers or detection kits used therein. The present invention is particularly useful in the diagnosis of hereditary diseases, analysis of nucleotide polymorphisms and the like.

### BACKGROUND ART

Nucleotide polymorphisms in genes are thought to be one cause of drug treatment failure and side effects related to drug metabolism in individuals. Nucleotide polymorphisms are also known to cause individual variations in the basal metabolism or constitution and the like. In addition, they also serve as genetic markers for various conditions. Consequently, analysis of such polymorphisms is clinically important, and routine phenotype classification is particularly recommended in clinical studies involving psychiatric patients and suicidal subjects (Gram and Brsen, ***European Consensus Conference on Pharmacogenetics,*** Commission of the European Communities, Luxembourg, 1990, pp. 87-96; Balant et al, ***Eur. J. Clin. Pharmacol.*** Vol. 36 pp. 551-554 (1989)). For such reasons there is demand for methods of analyzing nucleic acid sequences in order to detect the various genotypes once the responsible variant genes have been identified.

Conventional techniques of nucleic acid sequence analysis include for example the nucleic acid sequencing method. Sequencing can detect and identify nucleotide polymorphisms contained in a nucleic acid sequence, but considerable time and effort are required for template preparation, DNA polymerase reactions, polyacrylamide gel electrophoresis, nucleic acid sequence analysis and so forth. Automatic sequencers have simplified this process in recent years, but unfortunately expensive equipment is required.

Meanwhile, a variety of hereditary diseases are known to be caused by genetic point mutations, and in many cases it is known what point mutation of what site on the gene is responsible for the hereditary disease.

Conventional methods of detecting such anticipated point mutations include methods such as the PCR (polymerase chain reaction) method (Japanese Patent Publications Nos. H4-67960, H4-67957) in which genetic point mutations are detected using gene amplification. In this method, one of the pair of primers used in the gene amplification reaction is a wild-type primer, which perfectly complements the end region of the amplified region of the wild gene, and one is a variant primer, which perfectly complements the end region of the amplified region of the variant gene. The variant primer consists of nucleotides, which are complementary to nucleotides with the anticipated point mutation at the 3' terminus. Using these wild type and variant primers separately, sample genetic material is subjected to gene amplification.

If the sample genetic material is of the wild type, amplification of the nucleic acid occurs when the wild-type primer is used, while if the variant primer is used, there is no elongation reaction and amplification of the nucleic acid does not occur because the primer's 3' end is non-complementary (mismatched) to the corresponding nucleotides of the sample genetic material. On the other hand, if the sample genetic material is of the variant type, amplification occurs when the variant primer is used, but not when the wild-type primer is used. Consequently, it is possible to distinguish wild type from variant genetic material and thus to detect point mutations in the genetic material according to whether or not amplification occurs using either primer.

It is also possible to first amplify a nucleic acid sequence, which includes the point mutation, and then evaluate whether an elongation reaction occurs using the amplified nucleic acid in each primer. This method can also be applied to the analysis of nucleotide polymorphisms.

Following these principles, it would seem that nucleotide polymorphisms could be clearly detected using conventional methods, but the fact is that in the case of a point mutation the wild-type primer differs from the variant primer by only a single base unit, and some degree of reaction (that is, elongation or amplification) often occurs when a variant primer is used to elongate or amplify a wild-type gene or a wild-type primer is used to elongate or amplify a variant gene, making a clear determination difficult in many cases. Moreover, precise conditions such as the type of equipment used can affect whether or not elongation or amplification occurs using such a mismatched primer, so reproducibility is low. Consequently, operations are difficult because extremely precise control of temperature and other reaction conditions is necessary in order to ensure that no reaction occurs when the mismatched primer is used. Furthermore, when an elongation reaction is performed using a DNA polymerase such as Taq polymerase, which lacks 3' exonuclease activity, incorporation errors that occur during the elongation reaction cannot be corrected, and new errors may be included in an elongation reaction.

Using DNA polymerase having 3' exonuclease activity allows for accurate elongation reactions, but makes setting conditions even more difficult. In particular, when the 3' end sequence of the primer is mismatched an elongation reaction would normally not be expected, but may occur when a mismatched base is deleted by a correction function.

It is an object of the present invention to resolve the aforementioned issues and provide a method, which can clearly and reproducibly detect nucleotide polymorphisms in a nucleic acid sequence, and reagents therefore.

### DISCLOSURE OF THE INVENTION

With the foregoing in view, the inventors perfected the present invention when they discovered as a result of dedicated research that when wild-type primer is hybridized with variant genetic material or variant primer is hybridized with wild-type genetic material in the conventional method described above, an elongation or amplification reaction of the mismatched primer can be entirely prevented without precise control of the reaction conditions if the second nucleotide from the 3' end of the primer is made to correspond to the nucleotide polymorphism site.

Namely, the present invention comprises the following.
Item 1. A method of detecting a single nucleotide polymorphism contained in a nucleic acid sample, comprising the steps of
   (a) applying a wild-type primer and 1 or two types of variant primer either simultaneously or separately together with DNA polymerase to a chromosome or fragment thereof including a specific single nucleotide polymorphism site contained in a sample, and
   (b) detecting the nucleotide polymorphism contained in the nucleic acid sample according to whether or not the primers are elongated,
   wherein the second nucleotide from the 3' end of the primer corresponds to the respective nucleotides anticipated in the nucleotide polymorphism site.
Item 2. The detection method according to Item 1, being a method of detecting a single nucleotide polymorphism contained in a nucleic acid sample, comprising the steps of
   (a) applying a wild-type primer and 1 or two types of variant primer either simultaneously or separately together with DNA polymerase to a chromosome or fragment thereof including a specific single nucleotide polymorphism site contained in a sample, and
   (b) detecting the nucleotide polymorphism contained in the nucleic acid sample according to whether or not the primers are elongated,
   wherein the second base from the 3' end of the primer corresponds to the respective nucleotides anticipated in the nucleotide polymorphism site,
   and wherein at least one of the bases from the 3' end third position to the 5' end is replaced by a base not complementary to the base in the chain, which hybridizes µwith the primer in the chromosome or fragment.
Item 3. The detection method according to Item 2, wherein the 3' end third position base of each primer is replaced by a base not complementary to the base in the chain which hybridizes with the primer in the chromosome or fragment.
Item 4. The detection method according to Item 1, being a method of detecting a single nucleotide polymorphism contained in a nucleic acid sample, comprising the steps of
   (a) applying a wild-type primer and 1 or two types of variant primer either simultaneously or separately together with DNA polymerase to a chromosome or fragment thereof including a specific single nucleotide polymorphism site contained in a sample, and
   (b) detecting the nucleotide polymorphism contained in the nucleic acid sample according to whether or not the primers are elongated,
   wherein the second base from the 3' end of the primer corresponds to the respective nucleotides anticipated in the nucleotide polymorphism site,
   and wherein at least one of the bases from the 3' end third position to the 5' end is replaced by a base not complementary to the base in the chain which hybridizes with the primer in the chromosome or fragment, and said non-complementary base differs from primer to primer.
Item 5. The detection method according to Item 4, wherein the 3' end third position base of each primer is replaced by a base not complementary to the base in the chain which hybridizes with the primer in the chromosome or fragment, and said non-complementary base differs from primer to primer.
Item 6. The method according to Item 1, wherein the DNA polymerase has double-stranded DNA 3' exonuclease activity.
Item 7. The method according to Item 1, wherein the DNA polymerase is derived from ***Pyrococcus sp.*** KOD1 strain or ***Hyperthermophilic archaebacterium.***
Item 8. The method according to Item 1, including before step (a) a step of amplifying a chromosome or fragment thereof including a specific single nucleotide polymorphism site contained in a sample.
Item 9. The method according to Item 8, wherein the method of amplifying the chromosome or fragment is any one of the group consisting of PCR, NASBA, LCR, SDA, RCR and TMA.
Item 10. The method according to Item 1, wherein hybridization using a detection probe specific to the sequence of the elongation product of at least one of the group consisting of wild type and variant primers is used to detect whether or not each of the primers has been elongated.
Item 11. The method according to Item 10, wherein at least one of the primers or the detection probe is labeled in advance.
Item 12. The method according to Item 10, wherein at least one of the primers or the detection probe is labeled with at least one from the group consisting of enzymes, biotin, fluorescent material, hapten, antigen, antibodies, radioactive material and luminophore.
Item 13. The method according to Item 1, wherein step (a) is performed in a single tube, and hybridization using a detection probe specific to the sequence of the elongation product of at least one of the group consisting of wild-type and variant primers is used to detect whether or not each of the primers has been elongated.
Item 14. A method of detecting a single nucleotide polymorphism contained in a nucleic acid sample, comprising the steps of
   (a) applying a wild-type primer and 1 or two types of variant primer either simultaneously or separately together with DNA polymerase to a chromosome or fragment thereof including a specific single nucleotide polymorphism site contained in a sample, and
   (b) detecting the nucleotide polymorphism contained in the nucleic acid sample according to whether or not the chromosome or fragment containing the specific single nucleotide polymorphism site is amplified,
   wherein the second nucleotide from the 3' end of the primer corresponds to the respective nucleotides anticipated in the nucleotide polymorphism site.
Item 15. The method according to Item 14, being a method of detecting a single nucleotide polymorphism contained in a nucleic acid sample, comprising the steps of
   (a) applying a wild-type primer and 1 or two types of variant primer either simultaneously or separately together with DNA polymerase to a chromosome or fragment thereof including a specific single nucleotide polymorphism site contained in a sample, and
   (b) detecting the nucleotide polymorphism contained in the nucleic acid sample according to whether or not the chromosome or fragment containing the specific single nucleotide polymorphism site is amplified,
   wherein the second nucleotide from the 3' end of the primer corresponds to the respective nucleotides anticipated in the nucleotide polymorphism site,
   and wherein at least one of the bases from the 3' end third position to the 5' end is replaced by a base not complementary to the base in the chain, which hybridizes with the primer in the chromosome or fragment.
Item 16. The detection method according to Item 15, wherein the 3' end third position base of each primer is replaced by a base not complementary to the base in the chain which hybridizes with the primer in the chromosome or fragment.
Item 17. The detection method according to Item 14, being a method of detecting a single nucleotide polymorphism contained in a nucleic acid sample, comprising the steps of
   (a) applying a wild-type primer and 1 or two types of variant primer either simultaneously or separately together with DNA polymerase to a chromosome or fragment thereof including a specific single nucleotide polymorphism site contained in a sample, and
   (b) detecting the nucleotide polymorphism contained in the nucleic acid sample according to whether or not the chromosome or fragment containing the specific single nucleotide polymorphism site is amplified,
   wherein the second base from the 3' end of the primer corresponds to the respective nucleotides anticipated in the nucleotide polymorphism site,
   and wherein at least one of the bases from the 3' end third position to the 5' end is replaced by a base not complementary to the base in the chain which hybridizes with the primer in the chromosome or fragment, and said non-complementary base differs from primer to primer.
Item 18. The detection method according to Item 17, wherein the 3' end third position base of each primer is replaced by a base not complementary to the base in the chain which hybridizes with the primer in the chromosome or fragment, and said non-complementary base differs from primer to primer.
Item 19. The method according to Item 14, wherein the DNA polymerase has double-strand DNA 3' exonuclease activity.
Item 20. The method according to Item 14, wherein the DNA polymerase is derived from ***Pyrococcus sp.*** KOD1 strain or ***Hyperthermophilic archaebacterium.***
Item 21. The method according to Item 14, wherein the method of amplifying the chromosome or fragment is any one of the group consisting of PCR, NASBA, LCR, SDA, RCR and TMA.
Item 22. The method according to Item 14, wherein hybridization using a detection probe specific to the sequence of the respective elongation products of the wild-type and/or variant primers is used to detect whether or not a chromosome or fragment containing the specific single nucleotide polymorphism site has been amplified.
Item 23. The method according to Item 22, wherein at least one of the primers or the detection probe is labeled in advance.
Item 24. The method according to Item 22, wherein at least one of the primers or the detection probe is labeled with at least one from the group consisting of enzymes, biotin, fluorescent material, hapten, antigen, antibodies, radioactive material and luminophore.
Item 25. The method according to Item 14, wherein step (a) is performed in a single container, and hybridization with detection probes specific to the sequences of the respective amplification products from the wild-type and/or variant primers is used to detect whether or not each of the primers has been elongated.
Item 26. A single nucleotide polymorphism detection reagent kit comprising a wild-type primer, one or two types of variant primer, DNA polymerase and 4 types of deoxynucleoside triphosphate (dNTP), wherein the second base from the 3' end of each primer corresponds to the respective nucleotides anticipated in the nucleotide polymorphism site.
Item 27. The kit according to Item 26, being a single nucleotide polymorphism detection reagent kit comprising a wild-type primer, one or two types of variant primer, DNA polymerase and 4 types of deoxynucleoside triphosphate (dNTP), wherein the second base from the 3' end of each primer corresponds to the respective nucleotides anticipated in the nucleotide polymorphism site, and wherein at least one of the bases from the 3' end third position to the 5' end is replaced by a base not complementary to the base in the chain which hybridizes with the primer in the chromosome or fragment.
Item 28. The kit according to Item 27, wherein the 3' end third position base of each primer is replaced by a base not complementary to the base of the chain which hybridizes with the primer in the chromosome or fragment.
Item 29. The kit according to Item 26, being a single nucleotide polymorphism detection reagent kit comprising a wild-type primer, one or two types of variant primer, DNA polymerase and 4 types of deoxynucleoside triphosphate (dNTP), wherein the second base from the 3' end of each primer corresponds to the respective nucleotides anticipated in the nucleotide polymorphism site, and wherein at least one of the bases from the 3' end third position to the 5' end is replaced by a base not complementary to the base in the chain which hybridizes with the primer in the chromosome or fragment, and wherein said non-complementary base differs from primer to primer.
Item 30. The kit according to Item 29, wherein the 3' end third position base in each primer is replaced by a base not complementary to the base of the chain which hybridizes with the primer in the chromosome or fragment, and wherein said non-complementary base differs from primer to primer.
Item 31. A single nucleotide polymorphism detection reagent kit comprising a wild-type primer, one or two types of variant primer, DNA polymerase, 4 types of deoxynucleoside triphosphate (dNTP) and a detection probe, wherein the second base from the 3' end of each primer corresponds to the respective nucleotides anticipated in the nucleotide polymorphism site.
Item 32. The kit according to Item 31, being a single nucleotide polymorphism detection reagent kit comprising a wild-type primer, one or two types of variant primer, DNA polymerase, 4 types of deoxynucleoside triphosphate (dNTP) and a detection probe, wherein the second base from the 3' end of each primer corresponds to the respective nucleotides anticipated in the nucleotide polymorphism site, and wherein at least one of the bases from the 3' end third position to the 5' end is replaced by a base not complementary to the base in the chain which hybridizes with the primer in the chromosome or fragment.
Item 33. The kit according to Item 32, wherein the 3' end third position base of each primer is replaced base not complementary to the base of the chain, which hybridizes with the primer in the chromosome or fragment.
Item 34. The kit according to Item 31, being a single nucleotide polymorphism detection reagent kit comprising a wild-type primer, one or two types of variant primer, DNA polymerase, 4 types of deoxynucleoside triphosphate (dNTP) and a detection probe, wherein the second base from the 3' end of each primer corresponds to the respective nucleotides anticipated in the nucleotide polymorphism site, and wherein at least one of the bases from the 3' end third position to the 5' end is replaced by a base not complementary to the base in the chain which hybridizes with the primer in the chromosome or fragment, and wherein said non-complementary base differs from primer to primer.
Item 35. The kit according to Item 34, wherein the 3' end third position base of each primer is replaced by a base not complementary to the base of the chain which hybridizes with the primer in the chromosome or fragment, and wherein said non-complementary base differs from primer to primer.

### BRIEF DESCRIPTION OF THE DRAWINGS

Figure 1 shows elongation or amplification in the detection method of the present invention.

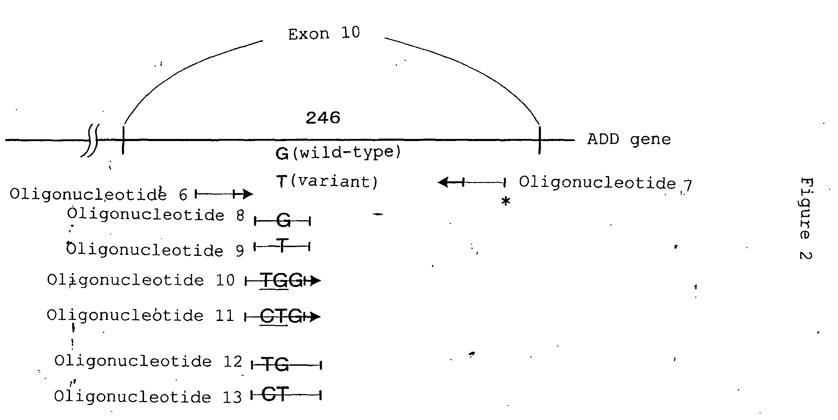

Figure 2 shows the positions of the oligonucleotides used in Example 2 of the present invention.

The present invention is explained in detail below.

In the present invention, the term "nucleotide polymorphism" signifies that a certain site on a chromosome or fragment has two or more possible bases depending on the individual, with one being the wild type and those with other bases being variant types. The term "nucleotide polymorphism site" signifies the position of a base polymorphism having both wild and variant types.

In the present invention, chromosomes or fragments thereof may be referred to simply as "nucleic acids". A "wild-type nucleic acid" is a nucleic acid containing a nucleotide polymorphism site in which the base at that site is of the wild type. A "variant nucleic acid" is a nucleic acid containing a nucleotide polymorphism site in which at least one and preferably one of the nucleotides in the wild-type nucleic acid has been replaced by another nucleotide, or a nucleic acid containing a partial inserted or deleted sequence, when the site of the nucleotide variation is known. More preferably, a "variant nucleic acid" is a nucleic acid containing a nucleotide polymorphism site in which a base other than the wild-type base has been substitute at that site. It is known that such nucleotide polymorphisms affect the constitution and the like, and the method of the present invention is a method of investigating whether or not nucleic acids in a sample have such anticipated variations.

A "wild-type primer" is a primer, which can hybridize with the nucleotide polymorphism site of the wild type. A "variant primer" is a primer, which can hybridize with a nucleotide polymorphism site of the variant type.

A "wild-type probe" is a probe, which specifically detects the elongated or amplified product of the wild-type primer. A "variant probe" is a probe, which specifically detects the elongated or amplified product of the variant primer.

There are no particular limitations on the chromosome or fragment thereof including a specific single nucleotide polymorphism site contained in a sample, as long as it comprises a target nucleic acid including a nucleotide polymorphism site which carries the desired genetic information, and mitochondria and the like are also allowed. Examples of such target nucleic acids include Alu sequences and the exons and introns of genes encoding proteins, as well as promoters and the like. More specific examples include genes related to drug metabolism, lifestyle disorders (high blood pressure, diabetes and the like) and various diseases including hereditary diseases. Examples include the ACE (Angiotensin I Converting Enzyme) gene in the case of high blood pressure and PPARγ (peroxisome proliferator-activated receptor γ) in the case of diabetes.

Nucleic acids are replicated by applying primer, 4 types of deoxynucleoside triphosphate (dNTP) and DNA polymerase to a target nucleic acid which has been denatured to a single strand, resulting in a primer elongation reaction with the target nucleic acid as the template and the synthesis of a complement strand to the nucleic acid sequence.

In the present invention, the method of detecting nucleotide polymorphisms in a nucleic acid sample according to whether or not the primer is elongated is a method of detecting polymorphisms by performing an elongation reaction in which a wild-type primer and one or two types of variant primer are applied either simultaneously or separately to a chromosome or fragment thereof including a specific single nucleotide polymorphism site contained in a nucleic acid sample.

Moreover, the method of detecting nucleotide polymorphisms in a nucleic acid sample according to whether or not the chromosome or fragment thereof containing a specific nucleotide polymorphism site is amplified is a method of detecting polymorphisms by performing an amplification reaction in which a wild-type primer corresponding to the forward primer and one or two types of variant primer are applied either simultaneously or separately together with a reverse primer to a nucleic acid sample containing a chromosome or fragment thereof which includes the specific single nucleotide polymorphism site.

The primers used in the present invention are designed so that the second nucleotide from the 3' end of the primer corresponds to the nucleotide of the nucleotide polymorphism sequence.

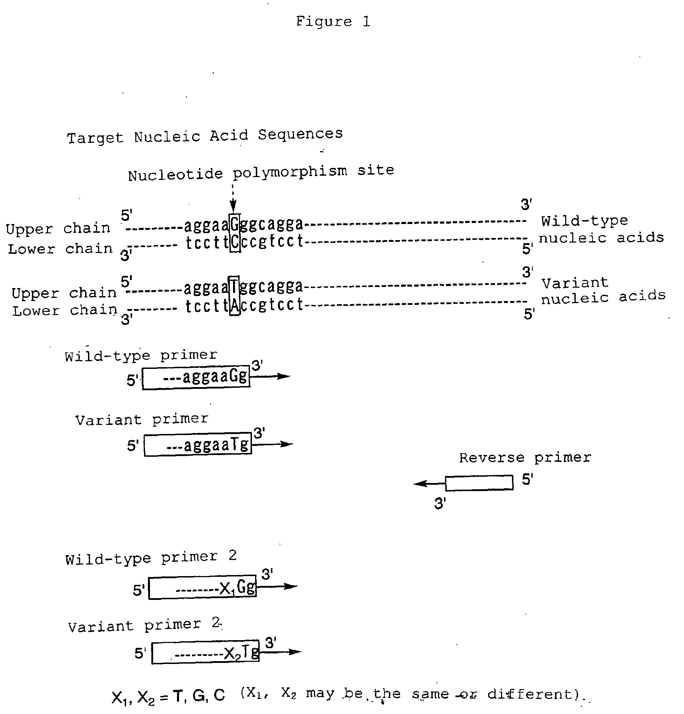

Namely, in Figure 1, if the wild-type base at a certain nucleotide polymorphism site is "G", the variant base is "T" and the 3' end base is "G", the wild-type primer for elongating or amplifying the wild-type nucleic acid is 5'-----Gg 3' and the variant primer is 5'-----Tg 3'. Using this design, combining wild-type primer with wild-type nucleic acid or variant primer with variant nucleic acid results in elongation or amplification because the primers perfectly match the respective nucleic acids. On the other hand, combining wild-type primer with variant nucleic acid or variant primer with wild-type nucleic acid does not result in elongation or amplification because the second base from the 3' end of the primer is non-complementary (mismatched). In particular, the inventors have discovered that when using DNA polymerase having 3' exonuclease activity a mismatched 3' end is recognized and deleted, but because the 3' end is complementary there is no exonuclease activity and no DNA polymerase reaction occurs.

Thus, in the method of the present invention, elongation or amplification of nucleic acid chains is entirely prevented in the case of wild-type primer/variant nucleic acid and variant primer/wild-type nucleic acid combinations, but occurs in the case of wild-type primer/wild-type nucleic acid and variant primer/variant nucleic acid combinations. Consequently, it is possible using the method of the present invention to clearly detect polymorphisms in target nucleic acids without the false positives that occur with conventional methods.

In addition to the aforementioned primers, the present invention also provides wild type and variant primers with one additional mismatch (see Wild-type primer 2 and Variant primer 2 in Figure 1). Namely, the aforementioned wild-type and variant primers used in the present invention are designed so that the second base from the 3' end of the primer corresponds to the nucleotide of the nucleotide polymorphism site, and may also be designed so that at least one of the bases from the 3' end third position to the 5' end is non-complementary to the base in the chain (the "lower chain" in Figure 1) which hybridizes with that primer in the chromosome or fragment (this site is referred to hereunder as the "artificial mismatch site"). In Wild-type primer 2 and Variant primer 2 in Figure 1, the 3' end third position base is shown as the artificial mismatch site, so that when the base of the chain (the "lower chain" in Figure 1) which hybridizes with the primer at that position is "T", the wild-type primer for elongating or amplifying the wild-type nucleic acids is 5'-----X₁Gg 3' and the variant primer is 5'-----X₂Tg 3' (where X₁ and X₂ may be any base other than the base A which complements T, namely T, G or C, and may be either the same or different).

Using this design, an elongation or amplification reaction occurs in combinations of wild-type primer/wild-type nucleic acid and variant primer/variant nucleic acid because the sequence of each primer matches at least through the second from the 3' end. In the case of a wild-type primer/variant nucleic acid or variant primer/wild-type nucleic acid combination, on the other hand, no elongation or amplification reaction occurs because the second base from the 3' end of the primer and one other base are artificially mismatched. Although DNA polymerase having 3' exonuclease activity acts to recognize and delete mismatches, the exonuclease is not active when the 3' end is complementary, and unwanted elongation reactions do not occur. We discovered that in this way, binding of wild-type primer with variant nucleic acids and variant primer without wild-type nucleic acids could be more effectively prevented.

Moreover, the primers of the present invention may also be wild-type and variant primers having a base sequence in which the second base from the 3' end corresponds to the anticipated nucleotide of the nucleotide polymorphism site, and at least one of the bases from the 3' end third position to the 5' end is not complementary to the base of the chain which hybridizes with the primer in the chromosome or fragment, and in which said non-complementary base differs from primer to primer. Namely, in this case X₁ and X₂ in Wild-type primer 2 and Variant primer 2 in Figure 1 have different bases.

For example, the primers are designed so that the second base from the 3' end is the anticipated nucleotide of the nucleotide polymorphism site, with the nucleotide of the wild-type primer corresponding to the nucleotide of the wild-type nucleic acid, and the nucleotide of the variant primer corresponding to the nucleotide of the variant nucleic acid. Moreover, if for example the base in the nucleic acid sequence of the chain which hybridizes with the primer is T at a given position between the 3' end third position and the 5' end of the primer, the wild-type primer is designed with T rather than A, and the variant primer with C or G rather than A or T. The pattern of these artificial mismatches is as follows.

**Table 1**

| Base of site designed with artificial mismatch (Base of chain which hybridizes with primer (lower chain in Figure 1)) | A | | | G | | | C | | | T | | |
|---|---|---|---|---|---|---|---|---|---|---|---|---|
| Base in wild-type primer | G | C | A | A | T | G | A | T | C | G | C | T |
| Base in variant primer | C A | G A | C G | G T | A G | A T | T C | A C | A T | C T | G T | G C |

Using such primers, not only is binding of wild-type primer with variant nucleic acid and variant primer with wild-type nucleic acid more effectively prevented, but detection is also more accurate because of the two-base difference resulting from the fact that in the detection probes mentioned below, the wild-type detection probe and variant detection probe differ by another base at the artificial mismatch site in addition to the nucleotide polymorphism site.

The length of the primers in the present invention is 13-35 bases or preferably 16-30 bases, and these primers have at least one artificial mismatch site as mentioned above. It may be located anywhere between the 3' end third position and the 5' end, with no particular restrictions, but a position close to the 3' end third position is preferred, and the 3' end third position is ideal. The chains that hybridize with the wild-type primer and variant primer may be either upper or lower chains.

When an artificial mismatch is used at the 3' end third position, and an elongation reaction is not expected from the nucleic acid used as the template, there is a continuous two-base mismatch including the base polymorphism site, and the elongation reaction is strongly blocked. When another site is used, however, the two noncontinuous mismatches in the primer block hybridization with the template, and the elongation reaction is suppressed as a result.

In principle, conventional methods can be used for the primer elongation method of the present invention. Ordinarily, elongation of primer with the target nucleic acid as the template is accomplished by applying a wild-type primer and 1 or 2 types of variant primer either simultaneously or separately together with 4 types of deoxynucleoside triphosphate (dNTP) and DNA polymerase to a chromosome or fragment thereof containing a specific polymorphism site denatured into a single chain.

This elongation reaction can be performed according to the methods described in ***Molecular Cloning, A Laboratory Manual*** (Sambrook et al, 1989). In the method of detecting nucleotide polymorphisms according to whether or not the primer has been elongated, if the target nucleic acids are not in sufficient quantity for detection, a nucleic acid fragment containing the polymorphism sequence can be amplified in advance by the amplification reaction shown below.

In the present invention, amplification of the chromosome or fragment thereof containing a specific nucleotide polymorphism site can also generally be accomplished by conventional methods, and is ordinarily accomplished between a reverse primer and a forward primer (wild-type or variant primer) with the target nucleic acid as the template, by applying a wild-type primer and 1 or 2 types of variant primer together with 4 types of deoxynucleoside triphosphate (dNTP), DNA polymerase and reverse primer to the chromosome or fragment thereof containing the specific nucleotide polymorphism site denatured to a single chain.

Possible methods of nucleic acid amplification include PCR, NASBA (Nucleic acid sequence-based amplification method; ***Nature*** Vol. 350 p. 91 (1991)), LCR (International Publication WO89/12696, Japanese Patent Application Laid-open No. H2-2934), SDA (Strand Displacement Amplification; ***Nucleic Acid Research*** Vol. 20 p. 1691 (1992)), RCR (International Publication WO90/1069), and TMA (Transcription Mediated Amplification Method. ***J. Clin. Microbiol.*** Vol. 31 p. 3270 (1993)).

Of these, the PCR method is a method of exponentially amplifying a region of the sample nucleic acids sandwiched between a pair of primers by cyclically repeating the three steps of denaturing, annealing and elongation in the presence of the sample nucleic acid, 4 types of deoxynucleoside triphosphate, the pair of primers and heat-resistant DNA polymerase. That is, the sample nucleic acids are denatured in the denaturing process, then in the annealing process the primers are hybridized with their respective complementary regions on the single-strand target nucleic acid, after which in the elongation process DNA strands complementary to each single-strand sample nucleic acid template are elongated by the action of DNA polymerase using each primer as the starter to produce double-strand DNA. In one such cycle, a single piece of double-strand DNA is amplified to two pieces of double-strand DNA. Consequently, by repeating this cycle n times such a region of sample DNA sandwiched between a pair of primers can in theory be amplified 2ⁿ times. Because the amplified DNA region exists in large quantities, it can easily be detected by a method such as electrophoresis. Therefore, gene amplification makes it possible to detect extremely small quantities (even one molecule) of a sample nucleic acid, which could not have been detected by conventional methods, and this technology has recently become very popular.

In the method of the present invention of detecting nucleotide polymorphisms according to whether or not a chromosome or fragment thereof containing a specific single nucleotide polymorphism site has been amplified, gene amplification is accomplished using, either separately or simultaneously, a wild-type primer, which can amplify wild-type nucleic acid, and a variant primer, which can amplify variant nucleic acid.

When a wild-type primer is used to elongate or amplify a target nucleic acid, the target nucleic acid is elongated or amplified if it is of the wild type but not if it is of the variant type. Conversely, when a variant primer is used to elongate or amplify a target nucleic acid, the target nucleic acid is elongated or amplified if it is of the variant type but not if it is of the wild type. Consequently, it is possible to accurately determine whether a target nucleic acid is of the wild or variant type by splitting a single sample into two batches and investigating whether elongation or amplification reactions occur using the wild-type primer with one batch and the variant primer with the other. In particular, humans and other higher organisms inherit two forms of each gene, one from the mother and one from the father, and this method makes it possible to determine whether a particular gene is homozygous for the wild or variant type or heterozygous for both. In the latter case the wild-type nucleic acid (wild-type gene) and variant nucleic acid (variant gene) are both present, so elongation or amplification occurs regardless of whether the wild-type primer or variant primer is used.

In the elongation and amplification steps of the present invention, the wild type and variant primers may also be applied together with DNA polymerase in a single container. That is, the wild-type and variant primers are added simultaneously together with DNA polymerase to nucleic acid contained in a single sample, producing an elongation or amplification reaction, and wild-type and variant probes are hybridized with the elongation or amplification product as described below in order to determine whether the target nucleic acid is of the wild or variant type.

Use of the primers of the present invention permits accurate detection even under relatively lax conditions, without the need for extremely precise control of such conditions as temperature and concentration during the elongation or amplification reaction.

For example, in the past the temperature for annealing primer with target nucleic acids often had to be controlled with a precision of ± 0.1°C, but when using the primers of the present invention the accuracy of the detection results is unaffected even when the error is ± 1 °C or more.

### DNA Polymerase

The DNA polymerase used in the elongation or amplification reactions of the present invention may be DNA polymerase ordinarily used in such reactions.

Preferably it should have double-strand DNA 3' exonuclease activity. DNA polymerase such as that derived from ***Thermus aquaticus*** is often used in amplification reactions but lacks 3' exonuclease activity, so that the amplification reaction continues even when a complement chain has not been accurately synthesized for the template sequence, leaving the possibility that an unexpected mutation has been included in the amplified nucleic acid fragment.

More preferable is DNA polymerase derived from ***Pyrococcus sp.*** KOD1 strain or ***Hyperthermophilic archaebacterium,*** which provide highly accurate elongation reactions.

### Elongation or Amplification Conditions

The elongation or amplification conditions will differ depending on the primer sequences and DNA polymerase used, but ordinary conditions may be used without any particular restrictions. As mentioned above, the temperature for annealing each primer with the target nucleic acid in the present invention need not be controlled to a precision ± 0.1 °C, and errors of ± 1 °C or more are allowable.

### Detection

Ordinary methods may be employed as the method of detecting nucleotide polymorphisms from the product of the aforementioned elongation reaction or amplification reaction, with no particular limitations, and desirable methods include those employing for example nucleotide sequencing, hybridization or restriction endonuclease.

When using hybridization for example, detection can be accomplished by first labeling the various primers with enzymes, biotin, fluorescent material, hapten, antigen, antibodies, radioactive material and luminophore and the like, and detecting the label after the elongation or amplification reaction. The reverse primer may also be labeled. Alternatively, the detection probe shown below may also be labeled. These may also be labeled with one or two or more markers.

Examples of enzymes include alkaline phosphatase and peroxidase.

Examples of fluorescent material include FITC, 6-FAM, HEX, TET, TAMRA, Texas Red, Cy3 and Cy5.

Examples of hapten include biotin and the like.

Examples of radioactive material include ³²P, ³⁵S and the like.

Examples of luminophore include ruthenium and the like.

Examples of antigen include digoxigenin, and examples of antibodies include anti-digoxigenin.

The label may be bound to any position on the primer as long as it does not affect the primer elongation reaction, and preferably to the 5' site.

Specifically, wild type and variant-type detection probes which can specifically capture the elongated or amplified products of the wild type and variant primers are bound to a microtitre plate or other solid phase by well-known methods. Next, the elongated or amplified products are denatured and added to the microtitre plates with the bound detection probes. The elongated or amplified product of the wild-type primer binds only to the wild-type detection probe, not to the variant detection probe. Similarly, the elongated or amplified product of the variant primer binds only to the variant detection probe, not to the wild-type detection probe. Finally, the nucleotide polymorphism in the chromosome or DNA fragment contained in the sample can be detected by detecting the label bound to the primer.

The wild type or variant detection probes may be the same, or may be specific to the elongated or amplified products of the wild type and variant primers, respectively.

In the method of the present invention, when the wild-type and variant primers differ by an artificial mismatch site in addition to the nucleotide polymorphism site, the wild-type detection or variant detection probes should be designed so that they can hybridize specifically with both the nucleotide polymorphism site and the artificial mismatch site. In this way, because a difference of two bases is observable between the wild-type and variant detection probes, their respective elongation/amplification products can be detected more clearly than if they differed by only the nucleotide polymorphism site. Consequently, the elongation/amplification reaction can be accomplished simultaneously with both primers without dividing the sample into two batches for the wild and variant type, and specific measurement is easy with the wild type and variant detection probes. In detail, wild type and variant primers are applied simultaneously together with DNA polymerase to nucleic acids contained in a single sample, resulting in an elongation or amplification reaction. By then denaturing the resulting product, dividing it into two and hybridizing it with wild-type and variant probes, it is possible to investigate whether the target nucleic acids are of the wild or variant type. When different labels are used for the wild type and variant primer, wells on a single plate can be used.

The length of the detection probes is not particularly restricted, and may be about 10-100 bases or preferably about 15-50 bases or more preferably about 18-35 bases.

There are also no particular restrictions on the detection conditions, which may for example be in accordance with the methods described in the ***Nihon Rinshokensa Jidokagakukai Kaishi (Journal of the Japanese Association for Automation of Clinical Testing)*** Vol. 20, Page 728 (1995).

### Kit

A kit in the present invention is a nucleotide polymorphism detection reagent kit containing wild-type primer, 1 or 2 kinds of variant primer, DNA polymerase and 4 types of deoxynucleoside triphosphate (dNTP), wherein in the wild-type and 1 or more types of variant primer,
- the second base from the 3' end of the primer corresponds to the respective nucleotides anticipated in the nucleotide polymorphism site,
- the second base from the 3' end of the primer corresponds to the respective nucleotides anticipated in the nucleotide polymorphism site, and at least one of the bases from the 3' end third position to the 5' end is replaced by a base not complementary to the base in the chain which hybridizes with the primer in the chromosome or fragment, or
- the second base from the 3' end of the primer corresponds to the respective nucleotides anticipated in the nucleotide polymorphism site, and at least one of the bases from the 3' end third position to the 5' end is replaced by a base not complementary to the base in the chain which hybridizes with the primer in the chromosome or fragment, and said non-complementary base differs from primer to primer.

When detecting by means of amplification, a reverse primer may also be included.

When probes are used for detection, the kit of the present invention may also include detection probes.

Detection probes may be used in the present invention, which are capable of detecting both wild and variant types, but preferably different types of detection probe should be prepared for detecting the wild and variant types, respectively. In this case, each detection probe should include the nucleotide polymorphism site.

Moreover, when at least one of the bases from the 3' end third position to the 5' end of each primer used for elongation or amplification is replaced by a base not complementary to the base in the chain which hybridizes with the primer in the chromosome or fragment, and said non-complementary base differs from primer to primer, the wild-type or variant type detection probe should be designed so that it can specifically detect the elongation or amplification product of the wild-type or variant primer. This allows for more specific detection because the detection probes differ by one more base at the artificial mismatch site in addition to the nucleotide polymorphism site.

Moreover, as mentioned above each primer or detection probe may also be labeled in advance with of enzymes, biotin, fluorescent material, hapten, antigen, antibodies, radioactive material, luminophore and the like.

Thus, in the method of the present invention, elongation of nucleic acid chains is entirely prevented in combinations of wild-type primer/variant nucleic acid and variant primer/wild-type nucleic acid, while elongation of nucleic acid chains occurs in combinations of wild-type primer/wild-type nucleic acid and variant primer/variant nucleic acid. Consequently, it is possible with the method of the present invention to accurately detect nucleotide polymorphisms in target nucleic acids without the false positives that occur with conventional methods.

### Examples

The present invention is described in more detail below based on examples. Of course, the present invention is not limited by the following examples.

### Example 1 Detection of Nucleotide Polymorphisms in the ACE (Angiotensin Converting Enzyme) Gene

### (1) Synthesis of primers for detecting ACE gene #2350 polymorphisms

An oligonucleotide having the base sequence shown by Seq. No. 1 (referred to hereunder as Primer 1), an oligonucleotide having the base sequence indicated by Seq. No. 2 (referred to hereunder as Primer 2), an oligonucleotide having the base sequence indicated by Seq. No. 3 (referred to hereunder as Primer 3), an oligonucleotide having the base sequence indicated by Seq. No. 4 (referred to hereunder as Primer 4), and an oligonucleotide having the base sequence indicated by Seq. No. 5 (referred to hereunder as Primer 5) were synthesized by the phosphoamidite method using a 392 Perkin-Elmer DNA synthesizer. Synthesis was performed according to the manual, and the various oligonucleotides were deprotected overnight at 55 °C with ammonia water. The oligonucleotides were purified using a Perkin-Elmer OPC column.

Primer 1 has the sequence of human ACE gene wild-type nucleic acid, with the wild-type nucleotide (A) at the nucleotide polymorphism site, which is the 3' end; Primer 2 has the sequence of human ACE gene variant nucleic acid, with the variant nucleotide (G) at the nucleotide polymorphism site, which is the 3' end; Primer 3 has the sequence of human ACE gene wild-type nucleic acid, with the wild-type nucleotide (A) at the nucleotide polymorphism site, which is the second from the 3' end; and Primer 4 has the sequence of human ACE gene variant nucleic acid, with the variant nucleotide (G) at the nucleotide polymorphism site, which is the second from the 3' end. Primer 5 a reverse primer which matches any of Primers 1-4.

### (2) Analysis of ACE Gene Polymorphisms by PCR

Three types of DNA solution (wild-type homozygous (homo A), variant homozygous (homo G) and heterozygous) extracted from human leukocytes by the phenol chloroform method were used as the samples, to which the following reagents were added, and the ACE gene polymorphisms were analyzed under the following conditions.

### (a) Reagents and Amplification Conditions

A 25µl solution containing the following reagents was prepared.

| | |
|---|---|
| Any of Primers 1-4 | 5 pmol |
| Primer 5 | 5 pmol |
| x10 buffer | 2.5 µl |
| 2 mM dNTP | 2.5 µl |
| 25 mM MgSO₄ | 1 µl |
| KODplus DNA polymerase | 0.2 U |
| Extracted DNA solutions | 100 ng |

Amplification Conditions
94 °C, 2 minutes
94 °C for 15 seconds, 55 °C for 30 seconds, 68 °C for 30 seconds (35 cycles)
68 °C, 2 minutes

### (b) Extraction

The resulting PCR product was subjected to agarose gel electrophoresis according to ordinary methods and stained with ethidium bromide, and the band of the amplification product was detected. The results were as shown in Table 2 below.

**Table 2**

| Primer DNA sample | Primer 1 | Primer 2 | Primer 3 | Primer 4 |
|---|---|---|---|---|
| Wild-type homozygous (homo A) | + | + | + | - |
| Variant homozygous (homo G) | + | + | - | + |
| Heterozygous | + | + | + | + |
| + Amplified | | | | |
| - Not amplified | | | | |

As shown above, the genotype of a sample can be clearly distinguished with KODplus DNA polymerase and primers (Primers 3 and 4), which include the nucleotide polymorphism sequence at the second from the 3' site.

### Example 2 Detection of ADD (Alpha Adducin) Gene Nucleotide Polymorphisms (Gly460Trp)

### (1) Synthesis of primers for detecting polymorphism in exons 10 and 246 of the ADD gene

Oligonucleotides having the base sequences indicated by Seq. Nos. 6-13 (referred to hereunder as "Oligonucleotides 6-13" or "Oligo 6-13") were synthesized by the phosphoamidite method using a Perkin-Elmer 392 model DNA synthesizer. Synthesis was performed according to the manual, and the various oligonucleotides were deprotected overnight at 55 °C with ammonia water. The oligonucleotides were purified using a Perkin-Elmer OPC column.

Oligonucleotides 6 (Seq. No. 6) and 7 (Seq. No. 7) share the same nucleotide sequence (not including the polymorphism site) for wild-type(G)/variant(T), with 6 being the sense chain and 7 being the antisense chain, and both are used as primers in the elongation/amplification reaction (homologous to the sequence of human ADD gene). Oligonucleotides 8 (Seq. No. 8) and 9 (Seq. No. 9) are used as probes to detect the respective amplification products of Oligonucleotides 6 and 7, with Oligonucleotide 8 being used to detect the wild type (G) and Oligonucleotide 9 the variant type (T) (the sequence of Oligonucleotide 8 is homologous to the wild-type sequence of the human ADD gene Gly460Trp nucleotide polymorphism, and that of Oligonucleotide 9 to the variant sequence of the human ADD gene Gly460Trp nucleotide polymorphism). Oligonucleotide 10 (Seq. No. 10) has the wild-type (G) nucleotide sequence at the second base from the 3' end and an artificial mismatch (A→T) at the third base, while Oligonucleotide 11 (Seq. No. 11) has the variant (T) nucleotide sequence at the second base from the 3' end an artificial mismatch (A→C) at the third base, and both are used in combination with Oligonucleotide 7 as primers in the amplification reaction. Oligonucleotide 12 (Seq. No. 12) and Oligonucleotide 13 (Seq. No. 13) are used as sense chain probes to detect the amplification reactions of Oligonucleotides 10 and 7 and Oligonucleotides 11 and 7, respectively. Oligonucleotides 7, 10 and 11 are labeled for use as necessary. Furthermore, uridine having a linker arm in the 5^{th} position has been introduced at the 5' end of Oligonucleotides 8, 9 12 and 13 using the synthesis method disclosed in Japanese Patent Publication No. S60-500717.

### (2) Analysis of ADD gene polymorphism by PCR and hybridization

### 1. Amplification reaction by PCR

Three types of DNA solution (wild-type homozygous (homo G, G/G), variant homozygous (homo T, T/T) and heterozygous(G/T)) extracted from human leukocytes by the phenol chloroform method were used as the samples, to which the following reagents were added, and the ADD gene polymorphism was analyzed under the following conditions.

### (a) Reagents and amplification conditions

A 25 µl solution containing the following reagents was prepared.
KOD DNA Polymerase Reaction Solution

| | |
|---|---|
| Any of Oligonucleotides 6, 10 and/or 11 | 5 pmol |
| Oligonucleotide 7 (5' labeled with biotin) | 5 pmol |
| x10 buffer | 2.5 µl |
| 2 mM dNTP | 2.5 µl |
| 25 mM MgSO₄ | 1.2 µl |
| KOD-plus DNA polymerase | 0.2 U |
| Extracted DNA solutions | 100 ng |

Amplification Conditions
94 °C, 5 minutes
94 °C for 15 seconds, 60 °C for 30 seconds, 68 °C for 30 seconds (35 cycles)
68 °C, 2 minutes

### (b) Detection by Hybridization

Oligonucleotides 8, 9, 12 and 13 were each prepared to 2.5 pmol/ml of a solution of 50mM boric acid buffer (pH 10.0) and 100 mM MgCl₂, and 100 µl per well was added to polystyrene microplates (MicroFLUOR B, Dynatech) and left at room temperature for about 15 minutes to bind to the inner surfaces of the microtiter plates. These were then replaced with 0.1 pmol dNTP, 0.5% PVP (polyvinyl pyrolidone), 5 X SSC, then blocked for about 2 hours at room temperature to suppress non-specific reactions, and finally washed in 1 x SSC and dried.

The amplification reaction solutions described in (a) were diluted 10 times, the amplified DNA in the amplification reaction solutions was denatured with 0.3N NaOH, and 20 µl of amplification reaction solution per sample was added to 100 µl of a solution of 200 mM citrate-phosphate buffer (pH 6.0), 2% SDS (sodium dodecyl sulfate), 750 mM NaCl, 0.1% NaN₃ and poured into the aforementioned microtiter plates with bound detection capture probes. These were covered with fluid paraffin to prevent evaporation, and shaken for 30 minutes at 55 °C. In this way, the amplified ADD gene fragment was specifically captured on the microtiter plates by the fixed probes.

Next, 2 X SSC (pH 7.0), 1% SDS was substituted and covered in the same way with fluid paraffin to prevent evaporation, followed by shaking for 20 minutes at 55 °C. Next, 100 µl of a solution of streptoavidin (DAKO D0396) labeled with alkaline phosphatase and diluted 2000 times with a solution of 50 mM tris-hydrochloric acid buffer (pH 7.5), 1% BSA solution was substituted, and shaken for 15 minutes at 37 °C. This served to bind the alkaline phosphatase-labeled streptoavidin specifically to the biotin of the captured DNA. After washing three times in 250 µl of 50 mM tris-hydrochloric acid buffer (pH 7.5), 0.025% Tween 20 solution, 50 µl of the luminescent base of alkaline phosphatase, dioxetan compound (Trade name Lumiphos 480, Lumigen Co.) was added, and after warming for 15 minutes at 37 °C luminescence was measured in a dark room using a photon counter (Hamamatsu Photonics) (unit = kilocount/second, kcps).

All of these steps were performed automatically with an automated DNA probe measurement system (see the ***Nihon Rinshokensa Jidokagakukal Kaishi (Journal of the Japanese Association for Automation of Clinical Testing)*** Vol. 20, Page 728 (1995), and took about 2.5 hours.

**Table 3**

| Forward primer | Reverse primer | Sample | Wild-type detection probe | Variant detection probe | Ratio |
|---|---|---|---|---|---|
| | | | Oligo 8 (wild-type (G)) | Oligo 9 (wild-type (T)) | |
| Oligo 6 (shared) | Oligo 7 (shared) | G/G | 16.25 | 0.85 | 19.12 |
| | | G/T | 7.45 | 7.07 | 1.05 |
| | | T/T | 0.39 | 5.03 | 0.08 |

| | | | Oligo 12 (G) | Oligo 13 (T) | |
|---|---|---|---|---|---|
| Oligo 10 (wild-type (G)) | Oligo 7 (shared) | G/G | 18.11 | 0.05 | 362.20 |
| | | G/T | 15.33 | 0.11 | 139.36 |
| | | T/T | 0.13 | 0.20 | -- |
| Oligo 11 (variant (T)) | Oligo 7 (shared) | G/G | 0.30 | 0.07 | -- |
| | | G/T | 0.36 | 12.78 | 0.03 |
| | | T/T | 0.11 | 26.02 | 0.00 |
| Oligo 10 and Oligo 11 | Oligo 7 (shared) | G/G | 46.27 | 0.31 | 149.26 |
| | | G/T | 11.99 | 6.28 | 1.92 |
| | | T/T | 0.36 | 15.48 | 0.02 |
| (kcps) | | | | | |
| G/G: Wild-type homozygous | | | | | |
| G/T: Heterozygous | | | | | |
| T/T Variant homozygous | | | | | |

As shown above, we were able to clearly distinguish genotypes by amplifying either separately or together two primers with the polymorphic base in the second position from the 3' end and an artificial mismatch at the third position, and using two detection oligonucleotides, which were specific to the amplification products and differed from each other by two bases.

### INDUSTRIAL APPLICABILITY

As described above, a method of clearly and easily detecting nucleotide polymorphisms in target nucleic acids is provided by the present invention. Since the method of the present invention does not produce false positives, highly reproducible results were obtained without especially precise control of the gene amplification conditions, and evaluation results did not differ depending on the type of machine for example. The method of the present invention also makes it possible to distinguish homozygous and heterozygous conjugation, something that was difficult with conventional methods.

## Claims

1. A method of detecting a single nucleotide polymorphism contained in a nucleic acid sample, comprising the steps of
(a) applying a wild-type primer and one or two types of variant primer either simultaneously or separately together with DNA polymerase to a chromosome or fragment thereof including a specific single nucleotide polymorphism site contained in a sample, and
(b) detecting the nucleotide polymorphism contained in the nucleic acid sample according to whether or not the primers are elongated,
wherein the second nucleotide from the 3' end of the primer corresponds to the respective nucleotides anticipated in the nucleotide polymorphism site.

2. The method of detecting a single nucleotide polymorphism contained in a nucleic acid sample according to Claim 1, comprising the steps of:
(a) applying a wild-type primer and 1 or two types of variant primer either simultaneously or separately together with DNA polymerase to a chromosome or fragment thereof including a specific single nucleotide polymorphism site contained in a sample, and
(b) detecting the nucleotide polymorphism contained in the nucleic acid sample according to whether or not the primers are elongated,
wherein the second base from the 3' end of the primer corresponds to the respective nucleotides anticipated in the nucleotide polymorphism site, and
wherein at least one of the bases from the 3' end third position to the 5' end is replaced by a base not complementary to the base in the chain, which hybridizes with the primer in the chromosome or fragment thereof.

3. The detection method according to. Claim 2, wherein the 3' end third position base of each primer is replaced by a base not complementary to the base in the chain which hybridizes with the primer in the chromosome or fragment thereof.

4. The method of detecting a single nucleotide polymorphism contained in a nucleic acid sample according to Claim 1, comprising the steps of:
(a) applying a wild-type primer and 1 or two types of variant primer either simultaneously or separately together with DNA polymerase to a chromosome or fragment thereof including a specific single nucleotide polymorphism site contained in a sample, and
(b) detecting the nucleotide polymorphism contained in the nucleic acid sample according to whether or not the primers are elongated,
wherein the second base from the 3' end of the primer corresponds to the respective nucleotides anticipated in the nucleotide polymorphism site, and
wherein at least one of the bases from the 3' end third position to the 5' end is replaced by a base not complementary to the base in the chain which hybridizes with the primer in the chromosome or fragment thereof, and said non-complementary base differs from primer to primer.

5. The detection method according to Claim 4, wherein the 3' end third position base of each primer is replaced by a base not complementary to the base in the chain which hybridizes with the primer in the chromosome or fragment thereof, and said non-complementary base differs from primer to primer.

6. The method according to Claim 1, wherein the DNA polymerase has double-stranded DNA 3' exonuclease activity.

7. The method according to Claim 1, wherein the DNA polymerase is derived from ***Pyrococcus sp.*** KOD1 strain or ***Hyperthermophilic archaebacterium.***

8. The method according to Claim 1, including before step (a) a step of amplifying a chromosome or fragment thereof including a specific single nucleotide polymorphism site contained in a sample.

9. The method according to Claim 8, wherein the method of amplifying the chromosome or fragment is any one of the group consisting of PCR, NASBA, LCR, SDA, RCR and TMA.

10. The method according to Claim 1, wherein hybridization using a detection probes specific to the sequence of the elongation product of at least one of the group consisting of wild type and variant primers is used to detect whether or not each of the primers has been elongated.

11. The method according to Claim 10, wherein at least one of the primers or the detection probe is labeled in advance.

12. The method according to Claim 10, wherein at least one of the primers or the detection probe is labeled with at least one from the group consisting of enzymes,- biotin, fluorescent material, hapten, antigen, antibodies, radioactive material and luminophore.

13. The method according to Claim 1, wherein step (a) is performed in a single tube, and hybridization with a detection probe specific to the sequence of the elongation product of at least one of the group consisting of wild-type and variant primers is used to detect whether or not each of the primers has been elongated.

14. A method of detecting a single nucleotide polymorphism contained in a nucleic acid sample, comprising the steps of
(a) applying a wild-type primer and one or two types of variant primer either simultaneously or separately together with DNA polymerase to a chromosome or fragment thereof including a specific single nucleotide polymorphism site contained in a sample, and
(b) detecting the nucleotide polymorphism contained in the nucleic acid sample according to whether or not the chromosome or fragment containing the specific single nucleotide polymorphism site is amplified, and
wherein the second nucleotide from the 3' end of the primer corresponds to the respective nucleotides anticipated in the nucleotide polymorphism site.

15. The method of detecting a single nucleotide polymorphism contained in a nucleic acid sample according to Claim 14, comprising the steps of
(a) applying a wild-type primer and one or two types of variant primer either simultaneously or separately together with DNA polymerase to a chromosome or fragment thereof including a specific single nucleotide polymorphism site contained in a sample, and
(b) detecting the nucleotide polymorphism contained in the nucleic acid sample according to whether or not the chromosome or fragment containing the specific single nucleotide polymorphism site is amplified,
wherein the second nucleotide from the 3' end of the primer corresponds to the respective nucleotides anticipated in the nucleotide polymorphism site, and
wherein at least one of the bases from the 3' end third position to the 5' end is replaced by a base not complementary to the base in the chain, which hybridizes with the primer in the chromosome or fragment thereof.

16. The detection method according to Claim 15, wherein the 3' end third position base of each primer is replaced by a base not complementary to the base in the chain which hybridizes with the primer in the chromosome or fragment thereof.

17. The method of detecting a single nucleotide polymorphism contained in a nucleic acid sample according to Claim 14, comprising the steps of:
(a) applying a wild-type primer and 1 or two types of variant primer either simultaneously or separately together with DNA polymerase to a chromosome or fragment thereof including a specific single nucleotide polymorphism site contained in a sample, and
(b) detecting the nucleotide polymorphism contained in the nucleic acid sample according to whether or not the chromosome or fragment containing the specific single nucleotide polymorphism site is amplified,
wherein the second base from the 3' end of the primer corresponds to the respective nucleotides anticipated in the nucleotide polymorphism site, and
wherein at least one of the bases from the 3' end third position to the 5' end is replaced by a base not complementary to the base in the chain which hybridizes with the primer in the chromosome or fragment thereof, and said non-complementary base differs from primer to primer.

18. The detection method according to Claim 17, wherein the 3' end third position base of each primer is replaced by a base not complementary to the base in the chain which hybridizes with the primer in the chromosome or fragment thereof, and said non-complementary base differs from primer to primer.

19. The method according to Claim 14, wherein the DNA polymerase has double-strand DNA 3' exonuclease activity.

20. The method according to Claim 14, wherein the DNA polymerase is derived from ***Pyrococcus sp.*** KOD1 strain or ***Hyperthermophilic archaebacterium.***

21. The method according to Claim 14, wherein the method of amplifying the chromosome or fragment thereof is any one of the group consisting of PCR, NASBA, LCR, SDA, RCR and TMA.

22. The method according to Claim 14, wherein hybridization using a detection probe specific to the sequence of the respective elongation products of the wild-type and/or variant primers is used to detect whether or not a chromosome or fragment containing the specific single nucleotide polymorphism site has been amplified.

23. The method according to Claim 22, wherein at least one of the primers or the detection probe is labeled in advance.

24. The method according to Claim 22, wherein at least one of the primers or the detection probe is labeled with at least one from the group consisting of enzymes, biotin, fluorescent material, hapten, antigen, antibodies, radioactive material and luminophore.

25. The method according to Claim 14, wherein step (a) is performed in a single container, and hybridization with detection probes specific to the sequences of the respective amplification products from the wild-type and/or variant primers is used to detect whether or not the chromosome or fragment containing the specific single nucleotide polymorphism has been amplified.

26. A single nucleotide polymorphism detection reagent kit comprising a wild-type primer, one or two types of variant primer, DNA polymerase and 4 types of deoxynucleoside triphosphate (dNTP), wherein the second base from the 3' end of each primer corresponds to the respective nucleotides anticipated in the nucleotide polymorphism site.

27. The kit according to Claim 26, being a single nucleotide polymorphism detection reagent kit comprising a wild-type primer, one or two types of variant primer, DNA polymerase and 4 types of deoxynucleoside triphosphate (dNTP), wherein the second base from the 3' end of each primer corresponds to the respective nucleotides anticipated in the nucleotide polymorphism site, and wherein at least one of the bases from the 3' end third position to the 5' end is replaced by a base not complementary to the base in the chain which hybridizes with the primer in the chromosome or fragment thereof.

28. The kit according to Claim 27, wherein the 3' end third position base of each primer is replaced by a base not complementary to the base of the chain which hybridizes with the primer in the chromosome or fragment thereof.

29. The kit according to Claim 26, being a single nucleotide polymorphism detection reagent kit comprising a wild-type primer, one or two types of variant primer, DNA polymerase and 4 types of deoxynucleoside triphosphate (dNTP), wherein the second base from the 3' end of each primer corresponds to the respective nucleotides anticipated in the nucleotide polymorphism site, end wherein at least one of the bases from the 3' end third position to the 5' end is replaced by a base not complementary to the base in the chain which hybridizes with the primer in the chromosome or fragment thereof, and wherein said non-complementary base differs from primer to primer.

30. The kit according to Claim 29, wherein the 3' end third position base in each primer is replaced by a base not complementary to the base of the chain, which hybridizes with the primer in the chromosome, or fragment thereof, and wherein said non-complementary base. differs from primer to primer.

31. A single nucleotide polymorphism detection reagent kit comprising a wild-type primer, one or two types of variant primer, DNA polymerase, 4 types of deoxynucleoside triphosphate (dNTP) and a detection probe, wherein the second base from the 3' end of each primer corresponds to the respective nucleotides anticipated in the nucleotide polymorphism site.

32. The kit according to Claim 31, being a single nucleotide polymorphism detection reagent kit comprising a wild-type primer, one or two types of variant primer, DNA polymerase, 4 types of deoxynucleoside triphosphate (dNTP) and a detection probe, wherein the second base from the 3' end of each primer corresponds to the respective nucleotides anticipated in the nucleotide polymorphism site, and wherein at least one of the bases from the 3' end third position to the 5' end is replaced by a base not complementary to the base in the chain which hybridizes with the primer in the chromosome or fragment thereof.

33. The kit according to Claim 32, wherein the 3' end third position base of each primer is replaced by a base not complementary to the base of the chain which hybridizes with the primer in the chromosome or fragment.

34. The kit according to Claim 31, being a single nucleotide polymorphism detection reagent kit comprising a wild-type primer, one or two types of variant primer, DNA polymerase, 4 types of deoxynucleoside triphosphate (dNTP) and a detection probe, wherein the second base from the 3' end of each primer corresponds to the respective nucleotides anticipated in the nucleotide polymorphism site, and wherein at least one of the bases from the 3' end third position to the 5' end is replaced by a base not complementary to the base in the chain which hybridizes with the primer in the chromosome or fragment thereof, and wherein said non-complementary base differs from primer to primer.

35. The kit according to Claim 34, wherein the 3' end third position base of each primer is replaced by a base not complementary to the base of the chain, which hybridizes with the primer in the chromosome, or fragment thereof, and wherein said non-complementary base differs from primer to primer.


SEQUENCE LISTING

<110> TOYO BOSEKI KABUSHIKI KAISHA

<120> A METHOD FOR DETECTING SNPs

<130> P00-48

<140>
<141>

<150> JP P1999-351837
<151> 1999-12-10

<150> JP P2000-208794
<151> 2000-07-10

<160> 13

<170> PatentIn Ver. 2.1

<210> 1
<211> 19
<212> DNA
<213> Artificial Sequence

<220>
<223> Description of Artificial Sequence:primer

<400> 1
gacgaatgtg atggccaca                                                    19

<210> 2
<211> 19
<212> DNA
<213> Artificial Sequence

<220>
<223> Description of Artificial Sequence:primer

<400> 2
gacgaatgtg atggccacg                                                    19

<210> 3
<211> 20
<212> DNA
<213> Artificial Sequence

<220>
<223> Description of Artificial Sequence:primer

<400> 3
gacgaatgtg atggccacat                                                   20

<210> 4
<211> 20
<212> DNA
<213> Artificial Sequence

<220>
<223> Description of Artificial Sequence:primer

<400> 4
gacgaatgtg atggccacgt 20

<210> 5
<211> 22
<212> DNA
<213> Artificial Sequence

<220>
<223> Description of Artificial Sequence:primer

<400> 5
ttgatgagtt ccacgtattt cg 22

<210> 6
<211> 22
<212> DNA
<213> Artificial Sequence

<220>
<223> Description of Artificial Sequence:primer

<400> 6
agacaagatg gctgaactct gg                                                22

<210> 7
<211> 22
<212> DNA
<213> Artificial Sequence

<220>
<223> Description of Artificial Sequence:primer

<400> 7
cacaccttag tcttcgactt gg                                                22

<210> 8
<211> 19
<212> DNA
<213> Artificial Sequence

<220>
<223> Description of Artificial Sequence:probe

<400> 8
gaagggcaga atggaagca                                                    19

<210> 9
<211> 19
<212> DNA
<213> Artificial Sequence

<220>
<223> Description of Artificial Sequence:probe

<400> 9
gaatggcaga atggaagca 19

<210> 10
<211> 20
<212> DNA
<213> Artificial Sequence

<220>
<223> Description of Artificial Sequence:primer

<400> 10
gacgaagctt ccgaggatgg 20

<210> 11
<211> 20
<212> DNA

<213> Artificial Sequence

<220>
<223> Description of Artificial Sequence:primer

<400> 11
gacgaagctt ccgaggactg                                                    20

<210> 12
<211> 20
<212> DNA
<213> Artificial Sequence

<220>
<223> Description of Artificial Sequence:probe

<400> 12
cgaggatggg cagaatggaa                                                    20

<210> 13
<211> 20
<212> DNA
<213> Artificial Sequence

<220>
<223> Description of Artificial Sequence:probe

<400> 13
cgaggactgg cagaatggaa 20